(19) Europäisches Patentamt
European Patent Office
Office européen des brevets

(11) EP 0 788 473 B1

(12) EUROPÄISCHE PATENTSCHRIFT

(45) Veröffentlichungstag und Bekanntmachung des Hinweises auf die Patenterteilung:
**06.11.2002 Patentblatt 2002/45**

(21) Anmeldenummer: **95906316.5**

(22) Anmeldetag: **09.01.1995**

(51) Int Cl.[7]: **C07C 227/32**, C07C 229/48, C07C 271/24, C07C 69/608, C07C 233/58, C07D 453/04

(86) Internationale Anmeldenummer:
**PCT/EP95/00059**

(87) Internationale Veröffentlichungsnummer:
**WO 95/019337 (20.07.1995 Gazette 1995/31)**

(54) **NEUES HOCHENANTIOSELEKTIVES VERFAHREN ZUR HERSTELLUNG VON ENANTIOMERENREINEN CYCLOPENTAN- UND -PENTEN-BETA-AMINOSÄUREN**

NOVEL HIGH ENANTIO-SELECTIVE PROCESS FOR PRODUCING PURE ENANTIOMERIC CYCLOPENTANE AND CYCLOPENTENE-BETA-AMINO ACIDS

NOUVEAU PROCEDE HAUTEMENT ENANTIO-SELECTIF POUR LA FABRICATION DE BETA AMINO-ACIDES DE CYCLOPENTANE ET DE CYCLOPENTENE ENANTIOMERIQUEMENT PURS

(84) Benannte Vertragsstaaten:
**AT BE CH DE DK ES FR GB GR IE IT LI LU MC NL PT SE**
Benannte Erstreckungsstaaten:
**LT SI**

(30) Priorität: **13.01.1994 DE 4400749**

(43) Veröffentlichungstag der Anmeldung:
**13.08.1997 Patentblatt 1997/33**

(60) Teilanmeldung:
**02012474.9**

(73) Patentinhaber: **BAYER AG**
**51368 Leverkusen (DE)**

(72) Erfinder:
- **MITTENDORF, Joachim**
  **D-42113 Wuppertal (DE)**
- **AROLD, Hermann**
  **D-42113 Wuppertal (DE)**
- **FEY, Peter**
  **D-42111 Wuppertal (DE)**
- **MATZKE, Michael**
  **D-42113 Wuppertal (DE)**
- **MILITZER, Hans-Christian**
  **D-51467 Bergisch Gladbach (DE)**
- **MOHRS, Klaus-Helmut**
  **D-42113 Wuppertal (DE)**

(56) Entgegenhaltungen:
**EP-A- 0 571 870**

- **TETRAHEDRON: ASYMMETRY, Bd.4, Nr.5, 1993, OXFORD GB Seiten 1047 - 1050 P. RENOLD, CH. TAMM 'Stereoselective Hydrolysis of Substituted Cylopentane Diesters with Pig Liver Esterase (PLE)'**
- **JOURNAL OF ORGANIC CHEMISTRY, Bd.54, 1989, EASTON US Seiten 5115 - 5122 H. J. GAIS ET AL. 'Enzyme-Catalyzed Asymetric Synthesis. 8. Enantioselectivity of Pig Liver Esterase Catalysed Hydrolyses of 4-Substituted Meso Cyclopentane 1,2-Diesters'**
- **ANGEWANDTE CHEMIE. INTERNATIONAL EDITION, Bd.23, Nr.1, 1984, WEINHEIM DE Seiten 67 - 68 M. SCHNEIDER ET AL. 'Enzymatic Synthesis of Chiral Building Blocks from Prochiral meso-Substrates: Preparation of Methyl(hyd rogen)-1,2-cycloalkanedicarboxylates'**
- **CHEMICAL ABSTRACTS, vol. 118, no. 13, 29. März 1993, Columbus, Ohio, US; abstract no. 124085, A. ROSENQUIEST ET AL. Seite 762 ; & ACTA CHEM. SCAND., Bd.46, Nr.11, 1992 Seiten 1127 - 1129**
- **CHEMICAL AND PHARMACEUTICAL BULLETIN., Bd.35, Nr.6, 1987, TOKYO JP Seiten 2266 - 2271 Y. MORIMOTO ET AL 'Enzymes and Catalysts. I. Pig Liver Esterase-Catalyzed Hydrolysis of Heterocyclic Diesters'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **JOURNAL OF THE CHEMICAL SOCIETY, PERKIN TRANSACTIONS 1, 1987, LETCHWORTH GB Seiten 1053 - 1058 J. HIRATAKE ET AL. 'Enantiotopic-group Differentiation. Catalytic Asymmetric Ring-opening of Prochiral Cyclic Acid Anhydrides with Methanol, using Cinchona Alkaloids' in der Anmeldung erwähnt**
- **JOURNAL OF THE CHEMICAL SOCIETY, CHEMICAL COMMUNICATIONS, 1985, LETCHWORTH GB Seiten 1717 - 1719 J. HIRATAKE ET AL. 'Catalytic Asymmetric Induction from Prochiral Cyclic Acid Anhydrides using Cinchona Alkaloids' in der Anmeldung erwähnt**
- **TETRAHEDRON: ASYMMETRY, Bd.1, Nr.8, 1990, OXFORD GB Seiten 517 - 520 R. A. AITKEN, J. GOPAL 'Catalytic Asymmetric Ring-Opening of Bridged Tricyclic Anhydrides' in der Anmeldung erwähnt**

## Beschreibung

Die vorliegende Erfindung betrifft ein hochenantioselektives Verfahren zur Herstellung von enantiomerenreinen Cyclopentan- und -penten-β-Aminosäuren.

Aus den Publikationen J. Chem. Soc. Perkin Trans. I, 1987. 1053; Tetrahedron Asymm. 1990, 517 und J. Chem. Soc. Chem. Commun. 1985, 1717-1719 ist das Prinzip einer asymmetrischen Ringöffnung prochiraler Säureanhydride mit Methanol und katalytischen Mengen Cinchona-Alkaloiden bekannt. Die entsprechenden Halbester werden mit mäßigen Enantiomerenüberschüssen von 35 bis 67% d.Th. erhalten.

Gegenstand der Erfindung ist ein hochenantioselektives Verfahren zur Herstellung von enantiomerenreinen Cyclopentan- und -penten-β-Aminosäuren der allgemeinen Formel (I)

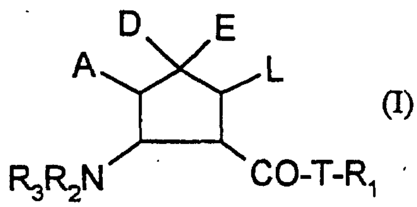

(I)

in welcher

A und L Wasserstoff bedeuten

oder

A und D oder E und L jeweils gemeinsam eine Doppelbindung bilden,

D und E gleich oder verschieden sind und für Wasserstoff, Halogen, Hydroxy oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^4R^5$ substituiert ist,
worin

$R^4$ und $R^5$ gleich oder verschieden sind und Wasserstoff, Phenyl oder geradkettiges oder verzweigtes Alkyl mit bis zu 6 Kohlenstoffatomen bedeuten,

oder

D und E gemeinsam fiir einen Rest der Formel

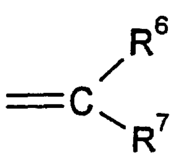

oder =N-OH stehen,
worin

$R^6$ und $R^7$ gleich oder verschieden sind und Wasserstoff, Halogen oder geradkettiges oder verzweigtes Alkyl, Alkoxy oder Oxyacyl mit jeweils bis zu 8 Kohlenstoffatomen, Benzyl oder Phenyl bedeuten,

oder

D und E zusammen fiir den Rest der Formel =O oder =S stehen,

$R^2$ für Wasserstoff oder
für eine Aminoschutzgruppe steht, oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
fiir geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel $-SO_2R^8$ steht,
worin

$R^8$ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^4R^5$ substitituiert sind,
worin

$R^4$ und $R^5$ die oben angegebene Bedeutung haben,

oder

für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^4R^5$ oder $-SO_2R^8$ substituiert ist,
worin

$R^4$, $R^5$ und $R^8$ die oben angegebene Bedeutung haben,

oder
für einen Aminosäurerest der Formel

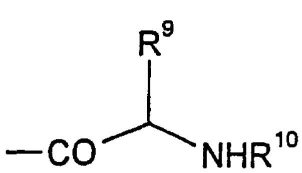

steht,
worin

$R^9$ Cycloalkyl mit 3 bis 8 Kohlenstoffatomen, Aryl mit 6 bis 10 Kohlenstoffatomen oder Wasserstoff bedeutet, oder
geradkettiges oder verzweigtes Alkyl mit bis zu 8 Kohlenstoffatomen bedeutet, wobei das Alkyl gegebenenfalls durch Cyano, Methylthio, Hydroxy, Mercapto, Guanidyl oder durch eine Gruppe der Formel $-NR^{11}R^{12}$ oder $R^{13}$-OC- substituiert ist,
worin

$R^{11}$ und $R^{12}$ unabhängig voneinander für Wasserstoff, geradkettiges oder verzweigtes Alkyl mit bis zu 8 Kohlenstoffatomen oder Phenyl stehen,

und

$R^{13}$ Hydroxy, Benzyloxy, Alkoxy mit bis zu 6 Kohlenstoffatomen oder die oben aufgeführte Gruppe $-NR^{11}R^{12}$ bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{11}R^{12}$ substituiert ist,
worin

$R^{11}$ und $R^{12}$ die oben angegebene Bedeutung haben,

und

$R^{10}$ Wasserstoff oder eine Aminoschutzgruppe bedeutet,

$R^3$ für Wasserstoff oder fiir geradkettiges oder verzweigtes Alkyl mit bis zu 8 Kohlenstoffatomen steht, das gegebenenfalls durch Phenyl substituiert ist,

oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

T für ein Sauerstoff- oder Schwefelatom oder für die -NH-Gruppe steht,

$R^1$ für Wasserstoff, für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch geradkettiges oder verzweigtes Alkyl, Acyl oder Alkoxycarbonyl mit jeweils bis zu 6 Kohlenstoffatomen oder durch eine Gruppe der Formel $-NR^4R^5$ oder $-SO_2R^8$ substituiert sind,
worin

$R^4$, $R^5$ und $R^8$ die oben angegebene Bedeutung haben,

oder für den Fall, daß T fiir die -NH-Gruppe steht,

$R^1$ für die Gruppe der Formel $-SO_2R^8$ steht,
worin

$R^8$ die oben angegebene Bedeutung hat,

dadurch gekennzeichnet, daß man meso-Dicarbonsäureanhydride der allgemeinen Formel (II)

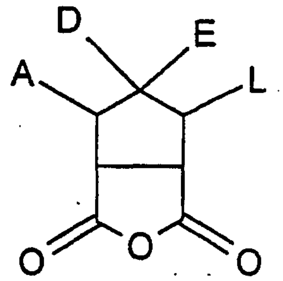

(II)

in welcher

A, D, E und L die oben angegebene Bedeutung haben,

durch eine asymmetrische Alkoholyse mit Alkoholen der allgemeinen Formel (III)

$$R^{15}\text{-OH} \quad \text{(III)}$$

in welcher

$R^{15}$ für geradkettiges oder verzweigtes Alkyl oder Alkenyl mit jeweils bis zu 5 Kohlenstoffatomen steht, die gegebenenfalls durch Cyano, Trimethylsilyl, Phenyl oder Trichlormethyl substituiert sind,

und in Anwesenheit equimolarer Mengen einer in enantiomerenreiner Form vorliegenden chiralen Aminbase, bezogen auf 1 mol der Dicarbonsäureanhydride der allgemeinen Formel (II), in inerten Lösemitteln, zunächst über die intermediäre, enantiomerenreine Salzstufe der allgemeinen Formel (IV)

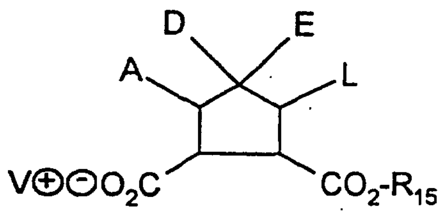

(+),(-) (IV)

in welcher

A, D, E, L und $R^{15}$ die oben angegebene Bedeutung haben

und

V für die chirale Aminbase steht,

in die enantiomerenreinen Verbindungen der allgemeinen Formel (IVa)

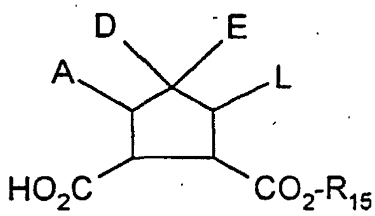

(+),(-) (IVa)

in welcher

A, D, E, L und $R^{15}$ die oben angegebene Bedeutung haben,

überführt,
anschließend nach Aktivierung der freien Carbonsäurefunktion durch Umsetzung mit flüssigem $NH_3$ die enantiomerenreinen Amide der allgemeinen Formel (V)

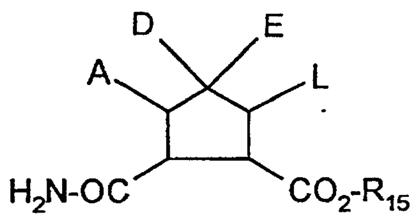

(+),(-) (V)

in welcher

A, D, E, L und $R^{15}$ die oben angegebene Bedeutung haben,

herstellt,
in einem weiteren Schritt durch Abspaltung des Restes $R^{15}$ in inerten Lösemitteln, enzymatisch oder in Anwesenheit eines Pd-Katalysators und jeweils in Abhängigkeit eines nucleophilen Hilfsmittels in die Verbindungen der allgemeinen Formel (VI) oder (VIa)

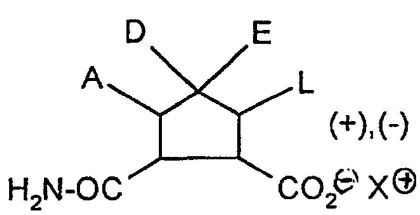

(+),(-) (VI)

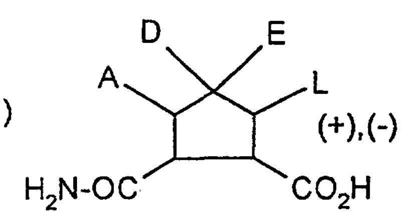

(+),(-) (VIa)

in welcher

A, D, E und L die oben angegebene Bedeutung haben,

und

X für ein Alkali- oder Erdalkaliatom, vorzugsweise für Natrium steht,

überführt,
und abschließend eine Hofmann-Umlagerung mit Alkali- oder Erdalkalihypochloriten in wäßriger Alkali- oder Erdalkalihydroxidlösung durchführt, in Lösung die freie Aminfunktion zunächst mit einer typischen Aminoschutzgruppe blokkiert und diese nach Isolierung der geschützten Verbindungen nach üblichen Bedingungen, unter Erhalt des jeweiligen reinen Enantiomers, nach üblichen Methoden abspaltet.

Das erfindungsgemäße Verfahren kann durch folgendes Formelschema beispielhaft erläutert werden:

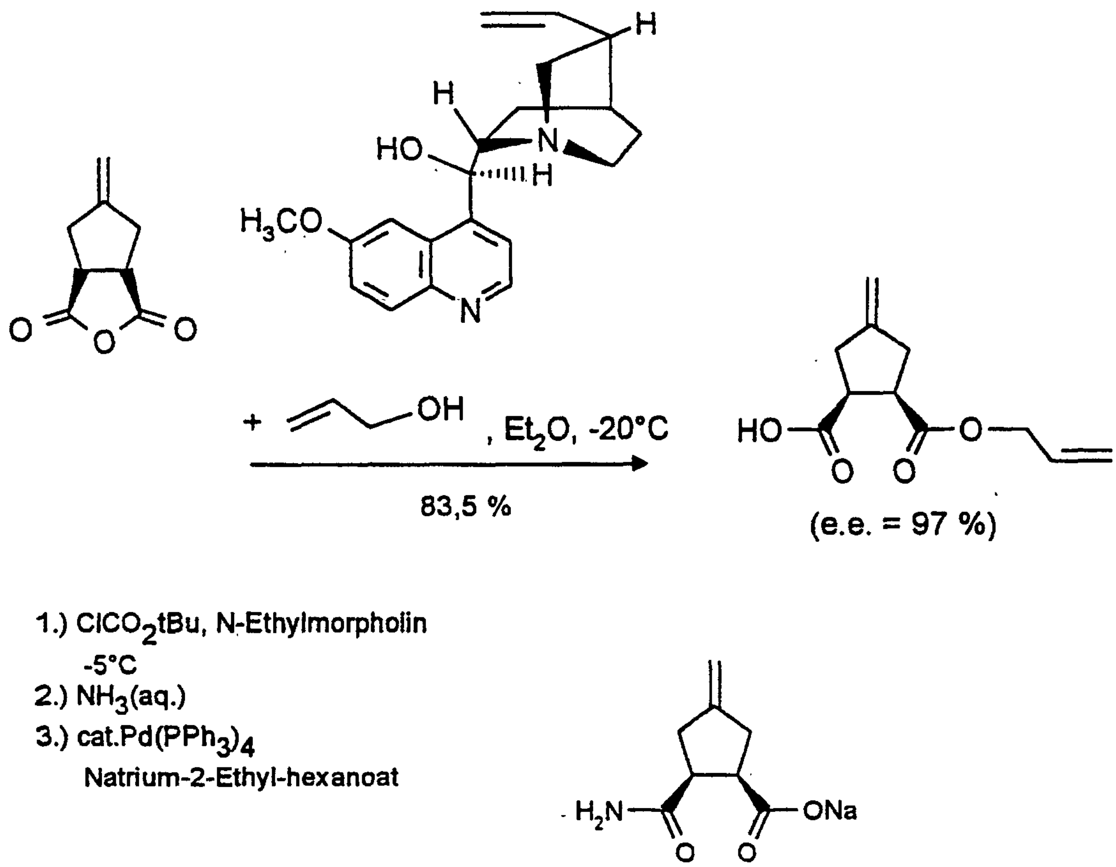

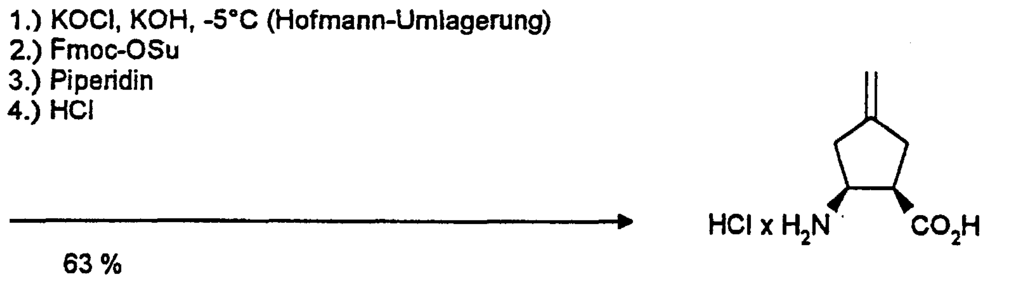


(-)-Enantiomer (e.e.≥ 98 %).

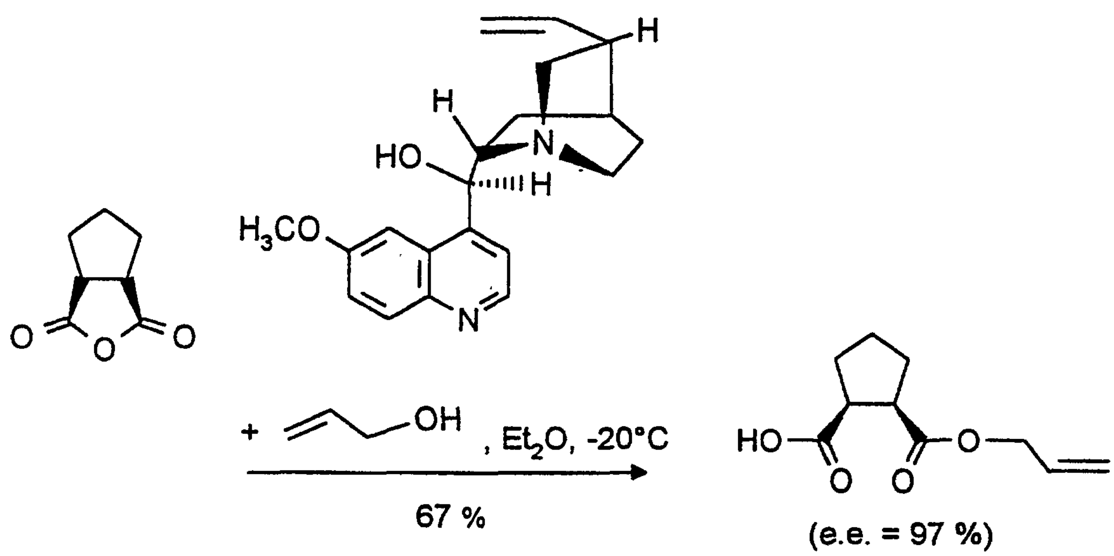


(e.e. = 97 %)

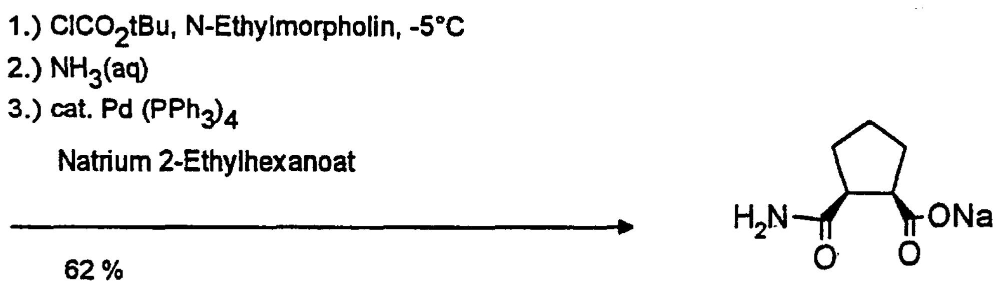


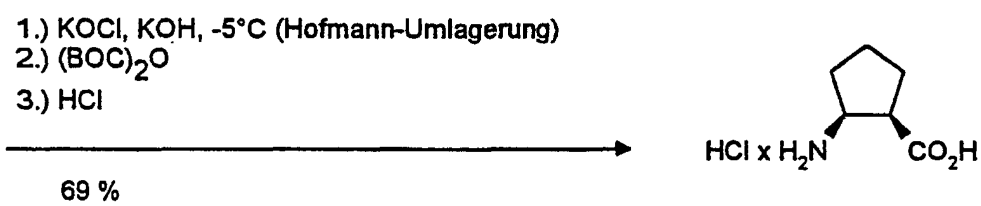


(-)-Enantiomer (e.e.≥ 98 %)

Überraschenderweise erhält man bei der Durchführung des erfindungsgemäßen Verfahrens die chiralen Verbindungen der allgemeinen Formel (I) auf elegante Weise mit sehr hoher Enantiomerenreinheit und gleichzeitig sehr guten Ausbeuten.

Im Gegensatz zum oben aufgeführten Stand der Technik ermöglicht das erfindungsgemäße Verfahren einen

hochenantioselektiven Weg zur Öffnung prochiraler Anhydride in Gegenwart von equimolaren Mengen einer chiralen Aminbase, wobei durch Kristallisation der intermediären Salze der entsprechenden Dicarbonsäuremonoester (Formel IVa) mit der chiralen Aminbase eine zusätzliche Enantiomerenanreicherung erfolgt. Bereits die Dicarbonsäuremonoester (Formal IVa) werden in einer guten Ausbeute und in hochreiner Form erhalten. Darüberhinaus zeichnet sich das erfindungsgemäße Verfahren im Gegensatz zum Stand der Technik dadurch aus, daß sowohl die chirale Aminbase durch eine einfache Extraktion mit verdünnten Säuren vollständig zurückgewonnen werden kann, als auch der in der Mutterlauge mit etwas geringerer Enantiomerenreinheit vorliegende Dicarbonsäuremonoester (Formal IVa) elegant in das entsprechende Anhydrid zurückgeführt werden kann.

Ein weiterer Vorteil des erfindungsgemäßen Verfahrens liegt, insbesondere auch im Hinblick auf den Kostenfaktor, darin, daß die gesamte Reaktionssequenz sehr kurz und wenig aufwendig ist und bereits die verschiedenen Zwischenstufen in sehr guten Ausbeuten und mit hoher Enantiomerenreinheit erhalten werden bzw. zurückgewonnen werden können.

Als Lösemittel für die Umsetzung der Dicarbonsäureanhydride der allgemeinen Formel (II) kommen alle inerten organischen Lösemittel in Frage, die sich unter den Reaktionsbedingungen nicht verändern. Hierzu gehören vorzugsweise Ether wie Diethylether, Dioxan, Diisopropylether, tert.Butylmethylether, Tetrahydrofuran oder Glykoldimethylether, oder Kohlenwasserstoffe wie Toluol, Benzol, Xylol, Hexan, Cyclohexan oder Erdölfraktionen, oder chlorierte Kohlenwasserstoffe, wie Chloroform oder Methylenchlorid, oder Amide wie Dimethylformamid, Dimethylacetamid oder Hexamethylphosphorsäuretriamid, oder Eisessig, Dimethylsulfoxid, Acetonitril oder Pyridin. Bevorzugt sind für die einzelnen Schritte Diisopropylether, Diethylether, Dioxan, tert.Butylmethylether und Toluol.

Die Reaktionstemperaturen können in einem größeren Bereich variiert werden. Im allgemeinen arbeitet man zwischen -60°C und +20°C, vorzugsweise zwischen -20°C und +25°C.

Die Umsetzungen können bei Normaldruck, aber auch bei erhöhtem oder erniedrigtem Druck (z.B. 0,5 bis 80 bar) durchgeführt werden. Im allgemeinen arbeitet man bei Normaldruck.

Als Alkohole (Formel III) für das erfindungsgemäße Verfahren eignen sich bevorzugt prim. Alkohole, wie beispielsweise Propanol, Butanol, Isopropanol, Ethanol, Allyl- oder Zimtalkohol.

Als chirale Aminbasen eignen sich für das erfindungsgemäßen Verfahren bevorzugt Alkaloide und Cinchona-Alkaloide. Besonders bevorzugt sind Cinchona-Alkaloide wie beispielsweise (+), (-)-Chinin, (+), (-)-Hydrochinin, (+), (-)-Cinchonidin, (+), (-)-Epichinidin, (+), (-)-Epicinchonidin, (+), (-)-Cinchonin, (+), (-)-Epicinchonin, (+), (-)-Epichinin, (+), (-)-Hydrochinidin, (+), (-) 4-Chlorbenzoat-Epichinin oder (+), (-) 4-Chlorbenzoat-Epicinchonin. Besonders bevorzugt sind (+), (-)-Chinin und (+), (-)-Chinidin.

Die chirale Aminbase wird in equivalenten Mengen bezogen auf 1 mol der Dicarbonsäureanhydride der allgemeinen Formel (II) eingesetzt.

Als Säuren für die Rückgewinnung der freien chiralen Aminbase eignen sich beispielsweise Mineralsäuren wie HCl, HBr oder Schwefelsäure.

Die Säure wird im allgemeinen in einer Menge von 1 mol bis 10 mol, bevorzugt von 1,5 mol bis 4 mol bezogen auf 1 mol der Verbindungen der allgemeinen Formel (IV) eingesetzt.

Die Rückgewinnung erfolgt im allgemeinen in einem Temperaturbereich von 0°C bis +50°C, bevorzugt von 20°C bis 30°C und Normaldruck.

Die Amidierung erfolgt im allgemeinen in inerten Lösemitteln in Anwesenheit einer Base und eines Aktivierungsreagenzes.

Als Lösemittel eignen sich hierbei inerte organische Lösemittel, die sich unter den angegebenen Reaktionsbedingungen nicht verändern. Hierzu gehören Ester wie Essigsäuremethyl-, ethyl-, isopropyl- oder n-butylester oder Ether wie Diethylether, Dioxan, Diisopropylether, tert.Butylmethylether, Tetrahydrofuran oder Glykoldimethylether, HalogenkohlenwasserstoffewieDichlormethan, Trichlormethan, Tetrachlormethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Essigsäuremethylester.

Als Basen für die Amidierung eignen sich organische Amine wie N-Ethylmorpholin, N-Methylmorpholin, Pyridin, Triethylamin oder N-Methylpiperidin.

Die Amidierung wird im allgemeinen in einem Temperaturbereich von -30°C bis +20°C, bevorzugt bei -20°C bis 0°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Aktivierungsreagenzien eignen sich Carbodiimide wie beispielsweise Diisopropylcarbodiimid, Dicyclohexylcarbodiimid oder N-(3-Dimethylaminopropyl)-N-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid, oder Alkylchloroformiate wie Ethyl- oder Isobutylchloroformiat, oder Benzotriazolyloxy-tris-(dimethylamino)phosphoniumhexafluorophosphat oder Phosphonsäurediphenylesteramid oder Methansulfonsäurechlorid,

gegebenenfalls in Anwesenheit von Basen wie Triethylamin oder N-Ethylmorpholin oder N-Methylpiperidin.

Die Base wird im allgemeinen in einer Menge von 1 mol bis 3 mol, bevorzugt von 1 mol bis 1,5 mol, bezogen auf 1 mol der Verbindungen der allgemeinen Formel (IVa) eingesetzt.

Die Abspaltung des Restes $R^{15}$ erfolgt im allgemeinen in inerten Lösemitteln wie beispielsweise in den oben aufgeführten Kohlenwasserstoffen, Estern oder Ethern, insbesondere in Tetrahydrofuran, Acetonitril, Dimethylformamid oder Ethylacetat. Bevorzugt ist Ethylacetat.

Als nucleophile Hilfsmittel für die Abspaltung des Restes $R^{15}$ eignen sich beispielsweise Carbonsäuren und deren Alkalimetallsalze (z.B. Ameisensäure, Essigsäure, 2-Ethylhexansäure, Natrium-2-ethyl-hexanoat), organische Amine wie beispielsweise Morpholin, Triethylamin, Pyrrolidin, Dimethyltrimethylsilylamin, Trimethylsilylmorpholin, n-Butylamin, Dimedon, Natrium-diethylmalonat, Tributylzirinhydrid, N,N-Dimethylbarbitursäure oder Ammoniumformiat. Bevorzugt sind 2-Ethylhexansäure und Natrium-2-ethyl-hexanoat.

Das Hilfsmittel wird im allgemeinen in einer Menge von 1 mol bis 20 mol, bevorvon 1,1 mol bis 2 mol, bezogen auf 1 mol der Verbindungen der allgemeinen Formel (V) eingesetzt.

Als Pd-Katalysatoren eignen sich im Rahmen des erfindungsgemäßen Verfahrens beispielsweise Tetrakistriphenylphosphinpalladium (O)(Pd($PPh_3)_4$/$PPh_3$, Palladium-dibenzylidenaceton ($Pd_2(dba)_3$), $Pd_2(dba)_3$ x $CHCl_3$, Pd $(dba)_2$, $PdCl_2$, $Pd(OAc)_2$, $PdCl_2(PhCN)_2$, $PdCl_2(CH_3CN)_2$ oder $PdCl_2(PPh_3)_2$. Bevorzugt sind Palladiumdibenzylidenaceton und Tetrakistriphenylphosphinpalladium.

Der Katalysator wird im allgemeinen in einer Menge von 0,0001 mol bis 0,2 mol, bevorzugt von 0,001 mol bis 0,05 mol, bezogen auf 1 mol der Verbindungen der allgemeinen Formel (V) eingesetzt.

Die Abspaltung des Restes $R^{15}$ wird im allgemeinen in einem Temperaturbereich von 0°C bis 60°C, bevorzugt von 20°C bis 30°C durchgeführt.

Im allgemeinen wird die Abspaltung bei Normaldruck durchgeführt. Es ist aber auch möglich, bei Unter- oder Überdruck (z.B. 0,5 bis 5 bar) zu arbeiten.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro--4,5-dimethoxybenzyloxycarbonyl, Methoxycarbonyl, Ethoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Phthaloyl, 2,2,2-Trichlorethoxycarbonyl, 2,2,2-Trichlortertbutoxycarbonyl, Menthyloxycarbonyl, 4-Nirrophenoxycarbonyl, N-Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt ist Fmoc.

Die Hofmann-Umlagerung der Verbindungen der allgemeinen Formel (VI) oder (VIa) erfolgt im allgemeinen mit Alkali- oder Erdalkalihypochloriten in wäßriger Alkali- oder Erdalkalihydroxidlösung. Bevorzugt ist Kaliumhypochlorit in wäßriger Kaliumhydroxidlösung.

Die Hofmann-Umlagerung erfolgt im allgemeinen in einem Temperaturbereich von -15°C bis +50°C, bevorzugt von -10°C bis +30°C und Normaldruck.

Die Einführung der Aminoschutzgruppe erfolgt nach üblichen Methoden in einem der oben aufgeführten Lösemittel, vorzugsweise Dioxan, in Anwesenheit einer Base, in einem Temperaturbereich von 0°C bis 60°C, vorzugsweise bei Raumtemperatur und Normaldruck.

Als Basen eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkali- und Erdalkalihydrogencarbonate wie Lithiumhydrogencarbonat, Natriumhydrogencarbonat, Kaliumhydrogencarbonat oder Bariumhydrogencarbonat, Alkali- oder Erdalkalicarbonate wie Natriumcarbonat, Kaliumcarbonat, oder Alkalialkoholate. Besonders bevorzugt ist Natriumhydrogencarbonat.

Die Base wird im allgemeinen in einer Menge von 1 mol bis 20 mol, bevorzugt von 5 mol bis 10 mol, bezogen auf 1 mol der Verbindungen der allgemeinen Formel (VI) eingesetzt.

Die Abspaltung der Aminoschutzgruppe erfolgt im allgemeinen mit den oben aufgeführten organischen Aminen. Bevorzugt ist Piperidin.

Die Base wird im allgemeinen in einer Menge von 1 mol bis 100 mol, bevorzugt von 20 mol bis 60 mol, bezogen auf 1 mol der geschützten Verbindung eingesetzt.

Die Abspaltung erfolgt im allgemeinen in einem Temperaturbereich von 0°C bis 60°C, bevorzugt von 20°C bis 30°C und Normaldruck.

Die Verbindungen der allgemeinen Formel (II) sind an sich bekannt oder nach publizierten Methoden herstellbar.

Die Alkohole der allgemeinen Formel (III) sind bekannt.

Die Verbindungen der allgemeinen Formeln (IV), und (V) sind neu und können beispielsweise wie oben beschrieben hergestellt werden.

Die Verbindungen der allgemeinen Formeln (VI) und (VIa) sind teilweise bekannt und können dann wie oben beschrieben hergestellt werden.

Bevorzugt werden nach dem erfindungsgemäßen Verfahren enantiomerenreine Verbindungen der allgemeinen Formel (I) hergestellt,
in welcher

A und L Wasserstoff bedeuten,

D und E gleich oder verschieden sind und für Wasserstoff,
Fluor, Chlor, Brom, Benzyl, Hydroxy oder für geradkettiges oder verzweigtes Alkyl mit bis zu 6 Kohlenstoffatomen stehen, das gegebenenfalls durch Halogen, Benzyloxy, Hydroxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ substituiert ist,
worin

$R^4$ und $R^5$ gleich oder verschieden sind und Wasserstoff oder geradkettiges oder verzweigtes Alkyl mit bis zu 4 Kohlenstoffatomen bedeuten,

oder

D und E gemeinsam für einen Rest der Formel

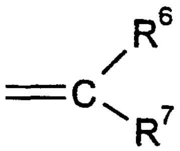

oder =N-OH stehen,
worin

$R^6$ und $R^7$ gleich oder verschieden sind und Wasserstoff, Fluor, Chlor, Brom,
oder geradkettiges oder verzweigtes Alkyl mit bis zu 6 Kohlenstoffatomen, Benzyl oder Phenyl bedeuten,

oder

D und E zusammen für den Rest der Formel =O oder =S stehen,

$R^2$ für Wasserstoff oder
für Boc, Benzyl, Benzyloxycarbonyl, Allyloxycarbonyl oder 9-Fluorenylmethyloxycarbonyl (Fmoc) steht,
oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substitutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder
für Benzoyl steht, das gegebenenfalls wie oben beschrieben substituiert ist, oder
für eine Gruppe der Formel $-SO_2R^8$ steht,
worin

$R^8$ geradkettiges oder verzweigtes Alkyl mit bis zu 6 Kohlenstoffomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^4R^5$ substituiert sind,
worin

$R^4$ und $R^5$ die oben angegebene Bedeutung haben,

für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-NR^6R^7$ oder $-SO_2R^8$ substituiert ist,
worin

$R^6$, $R^7$ die oben angegebene Bedeutung haben,

oder
für einen Aminosäurerest der Formel

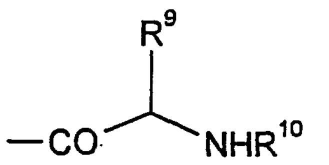

steht,
worin

$R^9$ Wasserstoff, geradkettiges oder verzweigtes Alkyl mit bis zu 6 Kohlenstoffatomen oder Benzyl bedeutet

und

$R^{10}$ Wasserstoff, Benzyloxy, Fmoc oder tert. Butoxycarbonyl bedeutet,

$R^3$ für Wasserstoff oder für geradkettiges oder verzweigtes Alkyl mit bis zu 6 Kohlenstoffatomen oder Benzyl steht,

oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,

T für ein Sauerstoff- oder Schwefelatom oder für die-NH-Gruppe steht,

$R^1$ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ oder $-SO_2R^8$ substituiert sind,
worin

$R^4$, $R^5$ und $R^8$ die oben angegebene Bedeutung haben,

oder für den Fall, daß T für die -NH-Gruppe steht,

$R^1$ für die Gruppe der Formel $-SO_2R^8$ steht, worin

$R^8$ die oben angegebene Bedeutung hat.

Besonders bevorzugt werden nach dem erfindungsgemäßen Verfahren enantiomerenreine Verbindungen der allgemeinen Formel (I) hergestellt, in welcher

A und L Wasserstoff bedeuten,

oder

D und E gleich oder verschieden sind und für Wasserstoff, Fluor, Chlor, Brom, Benzyl, Hydroxy oder für geradkettiges oder verzweigtes Alkyl mit bis zu 4 Kohlenstoffatomen stehen, das gegebenenfalls durch Hydroxy oder Benzyloxy substituiert ist,

oder

D und E gemeinsam für einen Rest der Formel

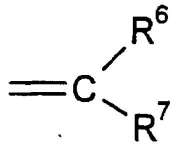

oder =N-OH stehen, worin

$R^6$ und $R^7$ gleich oder verschieden sind und Wasserstoff, Fluor, Chlor, Brom oder geradkettiges oder verzweigtes Alkyl mit bis zu 4 Kohlenstoffatomen oder Phenyl bedeuten, oder

D und E zusammen für den Rest der Formel =O oder =S stehen,

$R^2$ für Wasserstoff, Allyloxycarbonyl, Benzyl, Boc oder Fmoc steht, oder für geradkettiges oder verzweigtes Alkyl mit bis zu 4 Kohlenstoffatomen steht, oder für geradkettiges oder verzweigtes Acyl mit bis zu 4 Kohlenstoffatomen oder für eine Gruppe der Formel $-SO_2R^8$ steht, worin

$R^8$ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,

oder für einen Aminosäurerest der Formel

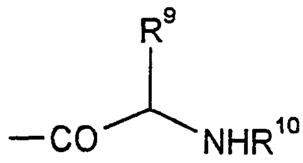

steht,
worin

$R^9$ Wasserstoff, geradkettiges oder verzweigtes Alkyl mit bis zu 4 Kohlenstoffatomen oder Benzyl bedeutet

und

$R^{10}$ Wasserstoff, tert. Butoxycarbonyl oder Fmoc bedeutet,

$R^3$ für Wasserstoff oder
für geradkettiges oder verzweigtes Alkyl mit bis zu 4 Kohlenstoffatomen steht,

oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 4 Kohlenstoffatomen bedeutet,

T für ein Sauerstoff- oder ein Schwefelatom oder für die -NH-Gruppe steht,

$R^1$ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel $-NR^4R^5$ oder $-SO_2R^8$ substituiert sind,
worin

$R^4$ and $R^5$ gleich oder verschieden sind und Wasserstoff, Methyl oder Ethyl bedeuten

und

$R^8$ die oben angegebene Bedeutung hat,

oder im Fall, daß T für die -NH-Gruppe steht,

$R^1$ für die Gruppe der Formel $-SO_2R^8$ steht,
worin

$R^8$ die oben angegebene Bedeutung hat.

Ganz besonders bevorzugt werden nach dem erfindungsgemäßen Verfahren enantiomerenreine Verbindungen der allgemeinen Formel (I) hergestellt,
in welcher

A, D, E und L für Wasserstoff stehen,

oder

A und L für Wasserstoff stehen

und

D und E gemeinsam eine Doppelbindung bilden.

Das erfindungsgemäße Verfahren ermöglicht auf hochenantioselektive Weise und gleichzeitig quantitativer Ausbeute den Zugang zu enantiomerenreinen Cyclopentan- und -penten-β-Aminosäuren der allgemeinen Formel (I), die wertvolle antimykotisch und antibakteriell wirksame Arzneimittel darstellen.

**Beispiel 1**

4-Methylen-cyclopentan-1,2-dicarbonsäure

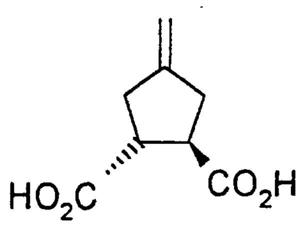

2,2245 kg (40 mol) Kaliumhydroxid werden in 15,7 l Wasser gelöst, auf Raumtemperatur gekühlt. 2,2263 kg (10 mol) 4-Methylen-1,2-cyclopentan-dicarbonsäurediethylester werden in 15,7 l Ethanol gelöst und bei Raumtemperatur zur Kaliumhydroxidlösung zulaufen lassen. Nach 30 Minuten Rühren bei RT wird das Ethanol am Rotationsverdampfer bei 55°C abdestilliert. Die verbleibende wäßrige Lösung wird 2 mal mit je 5 l Diethylether gewaschen, die Etherphase wird verworfen und die wäßrige Phase unter Kühlung mit 3 l konz. Salzsäure auf pH 2 eingestellt. Es wird 3 mal mit je 91 Essigester extrahiert, die Essigesterphase über Natriumsulfat getrocknet und bei 60°C am Rotationsverdampfer eingeengt.
Ausbeute: 1,66 kg; 97,5% d.Th.
Schmp.: 165-172°C

**Beispiel 2**

4-Methylen-cyclopentan-1,2-dicarbonsäureanhydrid

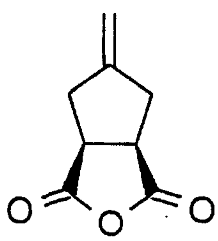

1325,6 g (7,79 mol) 4-Methylen-1,2-cyclopentan-dicarbonsäure und 6 l Propionsäureanhydrid werden 7 h unter Rückfluß (160°C) erhitzt. Ein Teil des Propionsäureanhydrids wird am Rotationsverdampfer bei 80°C abdestilliert und der Rückstand (2,165 kg) im Hochvakuum destilliert.
Ausbeute: 1014,9 g; 80,2% d.Th.
GC: 93,7%ig
Kp.: 97-100°C (0,5 mm Hg)

**Beispiel 3**

Cyclopentan-1,2-dicarbonsäureanhydrid

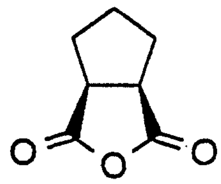

Die Herstellung erfolgt analog wie für Beispiel 2 beschreiben, ausgehend von 29,9 g (189 mmol) 1,2-Cyclopentan-dicarbonsäure.
Ausbeute: 17,8 g (77%)
Kp: 140°C (0,1 mbar, Kugelrohrdestillation)

**Beispiel 4 und Beispiel 5**

(-)-1,2-cis-4-Methylen-cyclopentan-1,2-dicarbonsäuremonoallylester-Chininsalz

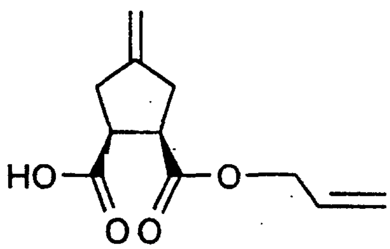

x Chinin

(-)-1,2-cis-4-Methylen-cyclopentan-1,2-dicarbonsäuremonoallylester

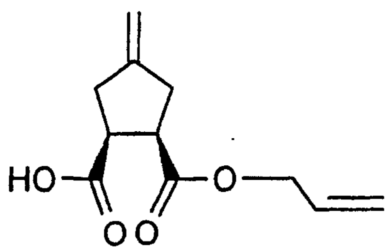

750 g (4,93 mol) 4-Methylen-1,2-cyclopentan-dicarbonsäureanhydrid werden in 34 l Diethylether gelöst und auf 0°C gekühlt. Es werden 1,6 kg (4,9 mol) (-)-Chinin zugegeben, auf -10°C gekühlt, 504,6 ml (7,4 mol) Allylalkohol zugegeben und 4 h bei -10°C bis -5°C gerührt, wobei Beispiel 4 ausfällt. Es wird abgesaugt, mit insgesamt 10 l Diethylether nachgewaschen und im Vakuum getrocknet. 2217,7 g der Verbindung aus Beispiel 4 werden in 30 l Essigester suspendiert und mit 10 l 1N Salzsäure gewaschen. Die vereinigten Salzsäurephasen werden 2 mal mit Essigester gewaschen, die vereinigten Essigesterphasen mit gesättigter Kochsalzlösung gewaschen, über Natriumsulfat getrocknet und am Rotationsverdampfer bei 50°C zu 859,2 g (83,5% d.Th.) eingeengt.
Enantiomerenüberschuß: ≥ 99% (HPLC, Chiracel OD)

$^1$H-NMR ($CDCl_3$): d = 2,59 - 2,91 (4H), 3,11 - 3,28 (2H), 4,58 (2H), 4,94 (2H); 5,18 - 5,37 (2H); 5,80 - 5,97 (1H).

Die wäßrige Salzsäurephase wird mit 2,5 M Natronlauge auf pH = 9,4 eingestellt, das ausgefallene Chinin wird abgesaugt, mit Wasser gewaschen und bei 50°C im Umlufttrockenschrank getrocknet.
Ausbeute:
Fp.: 160-162°C

**Beispiel 6**

(-)-Cis-cyclopentan-1,2-dicarbonsäuremonoallylester

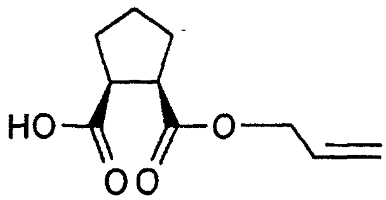

Die Herstellung erfolgt analog wie für Beispiel 4 und 5 beschrieben, ausgehend von 13,8 g (98,6 mmol) der Verbindung aus Beispiel 3.
Ausbeute: 13,0 g (67%)
$^1$H-NMR ($CDCl_3$): 1,56 - 2,20 (6H); 3,03 - 3,16 (2H); 4,08(2H); 5,69 - 5,90 (2H); 5,82 - 6,00 (1H).
Enantiomerenüberschuß ee = ≥ 98% (nach Kupplung der Carbonsäurefunktion mit L-Phenylglycinol mit HPLC bestimmt)

In Analogie zur Vorschrift der Beispiele 4, 5 und 6 werden die in Tabelle 1 aufgeführten Verbindungen hergestellt:

**Tabelle 1**

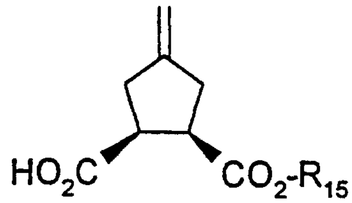

| Bsp.-Nr. | $R^{15}$ | Ausbeute (% d. Th.) | ee (%) |
|---|---|---|---|
| 7 | $-(CH_2)_2-CN^*$ | 41 | 78 |
| 8 | $-(CH_2)_2-Si(CH_3)_3^*$ | 87 | 64 |
| 9 | $-CH(CH_3)_2^*$ | 76 | 17 |
| 10 | $-C_2H_5^*$ | 74 | 88 |
| 11 | $-CH_3^*$ | 81 | 75 |
| 12 | $-(CH_2)_2CH_3$ | 72 | 98 |
| 13 | $-(CH_2)_3CH_3$ | 67 | 97,5 |
| 14 | $-CH=CH-C_6H_5$ | 82 | 94 |
| 15 | $-CH_2-CH(CH_3)_2$ | 69 | 95 |

* = in Toluol bei 0°C

**Beispiel 17**

(-)-1,2-cis-2-Aminocarbonyl-4-methylen-cyclopentan-1-carbonsäureallylester

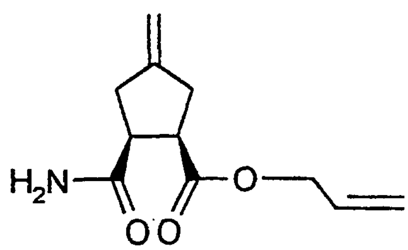

In 25 l Essigester werden 1,145 kg (5;44-7-mol)-{-)-1,2-cis-4-Methylen-1,2-cyclopentan-dicarbonsäuremonoallylester gelöst, es werden 729 ml (5,73 mol) N-Ethylmorpholin zugegeben und bei -6°C innerhalb 20 Minuten 743,5 ml (5,73 mol) Chlorameisensäureisobutylester zulaufen lassen. Es wird 1 h bei -6°C bis -10°C gerührt und bei dieser Temperatur 1276 ml (17,07 mol) einer vorgekühlten wäßrigen verdünnter Ammoniaklösung zulaufen lassen. Es wird

1 h bei dieser Temperatur gerührt, mit verdünnter Salzsäure auf pH 5 eingestellt, die Phasen werden getrennt, die wäßrige Phase wird mit 4 l Essigester gewaschen, die vereinigten Essigesterphasen werden 2 mal mit je 3 l gesättigter Natriumchloridlösung gewaschen, über Natriumsulfat getrocknet und am Rotationsverdampfer eingeengt. Durch Zugabe von 4 l Petrolether kristallisiert das Produkt aus, es wird abgesaugt, mit 4 l Petrolether verrührt, abgesaugt, mit 2 l Petrolether gewaschen und im Vakuum getrocknet.
Ausbeute: 996 g, 87,4% d.Th.
Smp.: 62°C

**Beispiel 18**

(-)-cis-2-Aminocarbonyl-cyclopentan-1-carbonsäureallylester

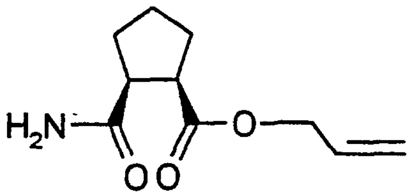

Die Herstellung erfolgt analog wie für Beispiel 17 beschrieben, ausgehend von 12,7 g (64 mmol) der Verbindung aus Beispiel 6.
Ausbeute: 9,9 g (78%)
Schmp.: 35°C
$[a]_D^{20}$ = -10,4 (c=1,05, $CHCl_3$)

**Beispiel 19**

(-)-1,2-cis-2-Aminocarbonyl-4-methylen-cyclopentan-1-carbonsäure Natriumsalz

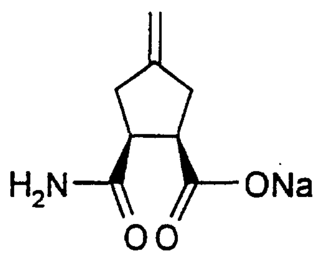

258 g (1,233 mol) (-)-1,2-cis-2-Aminocarbonyl-4-methylen-1,2-cyclopentancarbonsäureallylester werden unter Argonatmosphäre in 5 l Essigester gelöst. Es werden eine Lösung von 1,749 mol 2-Ethylhexansäure Natriumsalz in 3,15 l Essigester, 32,5 g (0,123 mol) Triphenylphosphin und 7,1 g (6,15 mmol) Tetrakis(triphenylphosphin)palladium zugegeben, und die Lösung 2 h bei RT gerührt, wobei das Produkt ausfällt. Die Suspension wird in 10 l Aceton ausgerührt, das Produkt wird abgesaugt und im Vakuum getrocknet.
Rohausbeute: 281,1 g; verunreinigt mit Ethylhexansäure Natriumsalz
$^1H$-NMR ($D_2O$) : d = 2,54 - 2,82 (4H); 3,08 - 3,25 (2H); 5,01 (2H)

**Beispiel 20**

(-)-1,2-cis-2-Aminocarbonyl-cyclopentan-1-carbonsäure Natriumsalz

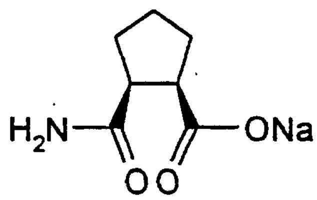

Die Herstellung erfolgt analog wie für Beispiel 19 beschrieben, ausgehend von 9,85 g (50,0 mmol) der Verbindung aus Beispiel 18.
Ausbeute: 7,1 g (79%)
$^1$H-NMR ($CDCl_3$): d = 1,52 - 2,14 (6H); 2,95 - 3,18 (2H).

**Beispiel 21**

(-)-1,2-cis-2-Aminocarbonyl-4-methylen-cyclopentan-1-carbonsäure

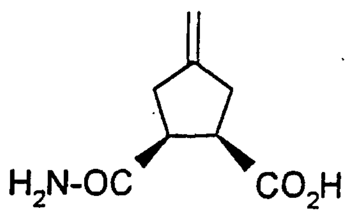

104,5 g (0,5 mol) (-)-1,2-cis-2-Aminocarbonyl-4-methylen-1,2-cyclopentancarbonsäureallylester werden unter Stickstoffatmosphäre in 1 l Essigsäure und 119 ml (0,75 mol) 2-Ethylhexansäure gelöst. Nach Zugabe von 13,1 g (0,05 mol) Triphenylphosphin und 3 g (0,005 mol) Bis(dibenzylidenaceton)palladium(O) wird die Reaktionslösung 5 h bei RT gerührt, wobei das Produkt auskristallisiert. Das Produkt wird abgesaugt, mit 50 ml Essigester gewaschen und im Vakuum getrocknet. Ausbeute: 63,4 g (75% d.Th.)

**Beispiel 22**

Wäßrige Lösung von (-)-1,2-cis-2-Amino-4-methylen-cyclopentan-carbonsäure

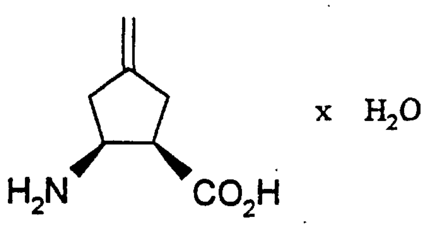

Zu einer Lösung von 478,45 g (8,54 mol) Kaliumhydroxid in 9 l Wasser werden bei 0°C 817 g (4,27 mol) (-)-1,2-cis-2-Aminocarbonyl-4-methylen-1,2-cyclopentancarbonsäure Natriumsalz zugegeben. Es werden 3,12 l einer 2,5

molaren Kaliumhypochloritlösung zugegeben und über Nacht bei dieser Temperatur gerührt. Die Lösung wird mit 5N Salzsäure auf pH 2 gestellt, 4 mal mit Diethylether gewaschen, die wäßrige Phase mit 5N Natronlauge auf pH 6,9 eingestellt und über Kieselgur abgesaugt.

**Beispiel 23**

Wäßrige Lösung von (-)-1,2-cis-2-Amino-cyclopentan-1-carbonsäure

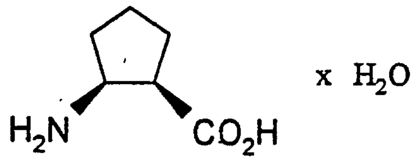

Die Herstellung erfolgt analog wie für Beispiel 22 beschrieben, ausgehend von 7,0 g (39,0 mmol) der Verbindung aus Beispiel 20.

**Beispiel 24**

(-)-1,2-cis-2-N-(9-Fluorenylmethoxycarbonyl)-amino-4-methylen-1-cyclopentancarbonsäure

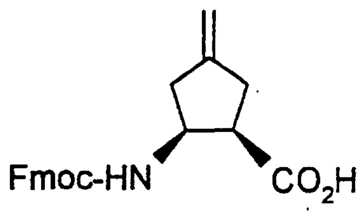

21,18 l (ca 3,7 mol) der wäßrigen Lösung von (-)-1,2-cis-2-Amino-4-methylen-cyclopentancarbonsäure werden mit 2,21 kg (26,34 mol) Natriumhydrogencarbonat versetzt und 15 Minuten bei RT gerührt. Es wird eine Lösung von 1,02 kg (3,01 mol) N-(9-Fluorenylmethoxycarbonyloxy)-succinimid in 5,4 l Dioxan zulaufen lassen und 5 h bei RT gerührt. Die Lösung wird filtriert, mit 20 l Diethylether gewaschen, die organische Phase wird mit verdünnter Natriumcarbonatlösung gewaschen und die wäßrigen basischen Produktphasen bei RT mit verd. Salzsäure auf pH 2 eingestellt. Es wird mehrfach mit Diethylether gewaschen, die vereinigten organischen Phasen werden über Natriumsulfat getrocknet und zu einem Öl am Rotationsverdampfer eingeengt.
Ausbeute: 1,47 kg; 104,6% d.Th.
$[a]_D^{20}$ = -18,8 (c=1, MeOH)
Smp.: 137°C
Enantiomerenüberschuß ee = > 99% (HPLC; Chiralpak AS)

**Beispiel 25**

(-)-1,2-cis-2-(tert.Butyloxycarbonyl)amino-cyclopentan-1-carbonsäure

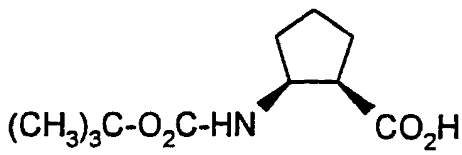

Zur neutralisierten wäßrigen Lösung der Verbindung aus Beispiel 23 gibt man 15 g Natriumcarbonat (pH = 9,8) und 200 ml Dioxan. Bei 0°C versetzt man mit 9,2 g (42 mmol) Di-tert.butyldicarbonat, läßt 10 min bei 0°C und 20 h bei Raumtemperatur rühren. Die Reaktionsmischung wird mit verdünnter Salzsäure auf pH 2 gestellt und dreimal mit jeweils 200 ml Essigester extrahiert. Die vereinten organischen Phasen werden mit ges. NaCl-Lösung gewaschen, über $Na_2SO_4$ getrocknet und am Rotationsverdampfer eingeengt. Der Rückstand wird an Kieselgel mit Dichlormethan/Methanol (20:1) als Eluens chromatographiert ($R_f$= 0,35).
Ausbeute: 7,34 g (82%)
$[a]_D^{20}$ = -35,0 (c=1,2, $CHCl_3$)

**Beispiel 26**

(-)-1,2-cis-2-Amino-4-methylen-cyclopentan-carbonsäure

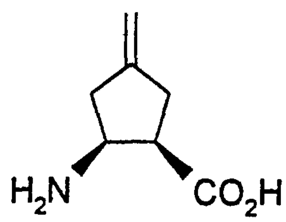

Eine Suspension von 1,47 kg (3,76 mol) (-)-1,2-cis-2-N-(9-Fluorenylmethoxycarbonyl)-amino-4-methylen-1-cyclopentan-carbonsäure in 13 l Piperidin wird 3 h bei Raumtemperatur bis zur klaren Lösung gerührt, anschließend werden im Dampfstrahlvakuum (60°C Badtemperatur) 8 l Piperidin abdestilliert, 10 l Diethylether zugegeben und über Nacht am Rotationsverdampfer gerührt. Die Suspension wird in einem Hobbock überführt, es werden 10 l Diethylether zugegeben, 2 h gerührt und das Produkt abgesaugt. Der Feststoff wird dreimal mit je 3 l Diethylether auf der Nutsche verrührt, trockengesaugt und 3 h bei Raumtemperatur im Hochvakuum über Phosphorpentoxid zu 377 g der freien Aminosäure getrocknet. Es wird in 6,6 l Ethanol/Wasser (9:1) klar gelöst und über Nacht auskristallisieren lassen. Das Produkt wird abfiltriert, mit 0,2 l 95%igem Ethanol nachgewaschen und im Vakuum über Phosphorpentoxid getrocknet.
Ausbeute: 214,5 g
Das Filtrat wird am Rotationsverdampfer eingeengt und wie oben beschrieben aus Ethanol/Wasser (9:1) kristallisiert.
Gesamtausbeute: 333,2 g; 63% d.Th.
Smp.: 222° C
$[a]_D^{20}$ = -31,6 (c=1, $H_2O$)

**Beispiel 27**

(-)-1,2-cis-2-Amino-4-methylen-cyclopentan-carbonsäure Hydrochlorid

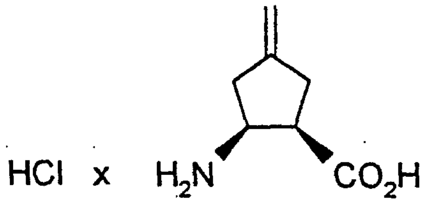

In 8 l bidestilliertem Wasser werden 341,7 g (2,42 mol) (-)-1,2-cis-2-Amino-4-methylen-cyclopentan-carbonsäure gelöst, über eine Glasinternutsche abgesaugt und die Nutsche mit 0,2 l bidestilliertem Wasser gewaschen. Die Lösung wird im Rotationsverdampferkolben mit 2,42 ml 1 N Salzsäure versetzt, zum Entfernen von Restlösemitteln bis zur beginnenden Kristallisation eingeengt (Bad 65°C), zweimal mit je 2 l bidestilliertem Wasser versetzt, bis zur Trockne eingeengt und 30 Minuten bei 40°C nachgetrocknet. Anschließend wird 84 h im Hochvakuum über Phosphorpentoxid und 24 h über Kaliumhydroxid getrocknet.
Ausbeute: 421,1 g; 97,7% d.Th.
$[a]_D^{20}$ = -11,6 (c=1, $H_2O$)

**Beispiel 28**

(-)-1,2-cis-2-Amino-cyclopentan-1-carbonsäure Hydrochlorid

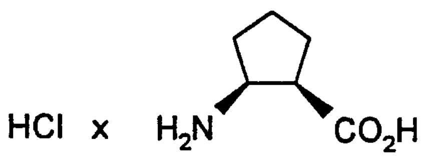

Eine Lösung der Verbindung aus Beispiel 25 (3,90 g, 17,0 mmol) in 30 ml 4 N HCl in Dioxan wird 2 h bei Raumtemperatur gerührt. Ausgefallenes Produkt wird abgesaugt, mit Dioxan und Ether gewaschen und im Hochvakuum 15 h getrocknet. Ausbeute: 2,39 g (84%)
$[a]_D^{20}$ = -5,7 (c=0,99, $H_2O$)
Enantiomerenüberschuß ee = ≥ 95% (HPLC, Chiralpak AS nach Überführung in die N-Fmoc geschützte Verbindung)

**Patentansprüche**

1. Verfahren zur Herstellung enantiomerenreiner Cyclopentan- und -penten-β-Aminosäuren der allgemeinen Formel (I)

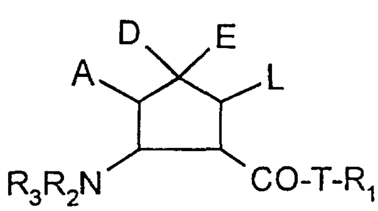

(I)

in welcher

A und L Wasserstoff bedeuten

oder

A und D oder E and L jeweils gemeinsam eine Doppelbindung bilden,

D und E gleich oder verschieden sind und für Wasserstoff, Halogen, Hydroxy oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^4R^5$ substituiert ist,
worin
$R^4$ und $R^5$ gleich oder verschieden sind und Wasserstoff, Phenyl oder geradkettiges oder verzweigtes Alkyl mit bis zu 6 Kohlenstoffatomen bedeuten,

oder

D und E gemeinsam für einen Rest der Formel

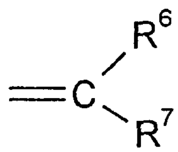

oder =N-OH stehen,
worin

$R^6$ und $R^7$ gleich oder verschieden sind und Wasserstoff, Halogen oder geradkettiges oder verzweigtes Alkyl, Alkoxy oder Oxyacyl mit jeweils bis zu 8 Kohlenstoffatomen, Benzyl oder Phenyl bedeuten,

oder

D and E zusammen für den Rest der Formel =O oder =S stehen,

$R^2$ für Wasserstoff oder
für eine Aminoschutzgruppe steht, oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel $-SO_2R^8$ steht,
worin

$R^8$ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^4R^5$ substitituiert sind,
worin

$R^4$ und $R^5$ die oben angegebene Bedeutung haben,

oder

für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^4R^5$ oder $-SO_2R^8$ substituiert ist,
worin

$R^4$, $R^5$ und $R^8$ die oben angegebene Bedeutung haben,

oder

für einen Aminosäurerest der Formel

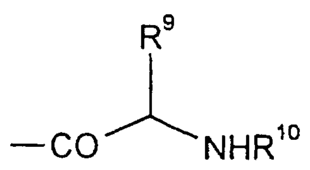

steht,
worin

$R^9$ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet, wobei das Alkyl gegebenenfalls durch Cyano, Methylthio, Hydroxy, Mercapto, Guanidyl oder durch eine Gruppe der Formel $-NR^{11}R^{12}$ oder $R^{13}$-OC- substituiert ist,
worin

$R^{11}$ und $R^{12}$ unabhängig voneinander für Wasserstoff, geradkettiges oder verzweigtes Alkyl mit bis zu 8 Kohlenstoffatomen oder Phenyl stehen,

und

$R^{13}$ Hydroxy, Benzyloxy, Alkoxy mit bis zu 6 Kohlenstoffatomen oder die oben aufgeführte Gruppe $-NR^{11}R^{12}$ bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{11}R^{12}$ substituiert ist,
worin

$R^{11}$ und $R^{12}$ die oben angegebene Bedeutung haben,

und

$R^{10}$ Wasserstoff oder eine Aminoschutzgruppe bedeutet,

$R^3$ für Wasserstoff oder für geradkettiges oder verzweigtes Alkyl mit bis zu 8 Kohlenstoffatomen steht,

das gegebenenfalls durch Phenyl substituiert ist,

oder

$R^2$ und $R^3$ gemeinsam für den Rest der Formel $=CHR^{14}$ stehen, worin

$R^{14}$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

T für ein Sauerstoff- oder Schwefelatom oder für die -NH-Gruppe steht,

$R^1$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch geradkettiges oder verzweigtes Alkyl, Acyl oder Alkoxycarbonyl mit jeweils bis zu 6 Kohlenstoffatomen oder durch eine Gruppe der Formel $-NR^4R^5$ oder $-SO_2R^8$ substituiert sind, worin

$R^4$, $R^5$ und $R^8$ die oben angegebene Bedeutung haben,

oder für den Fall, daß T für die -NH-Gruppe steht,

$R^1$ für die Gruppe der Formel $-SO_2R^8$ steht, worin

$R^8$ die oben angegebene Bedeutung hat,

**dadurch gekennzeichnet, daß** man meso-Dicarbonsäureanhydride der allgemeinen Formel (II)

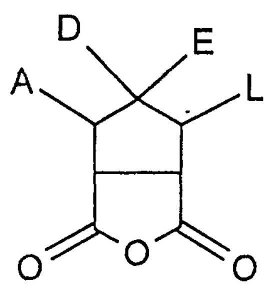

(II)

in welcher

A, D, E und L die oben angegebene Bedeutung haben,

durch eine asymmetrische Alkoholyse mit Alkoholen der allgemeinen Formel (III)

$$R^{15}\text{-OH} \quad \text{(III)}$$

in welcher

$R^{15}$ für geradkettiges oder verzweigtes Alkyl oder Alkenyl mit jeweils bis zu 5 Kohlenstoffatomen steht, die

gegebenenfalls durch Cyano, Trimethylsilyl, Phenyl oder Trichlormethyl substituiert sind,

und in Anwesenheit equimolarer Mengen einer in enantiomerenreiner Form vorliegenden chiralen Aminbase, bezogen auf 1 mol der Dicarbonsäureanhydride der allgemeinen Formel (II), in inerten Lösemitteln, zunächst über die intermediäre, enantiomerenreine Salzstufe der allgemeinen Formel (IV)

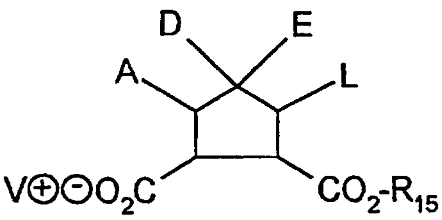

(+),(-) (IV)

in welcher

A, D, E, L und $R^{15}$ die oben angegebene Bedeutung haben

und

V für die chirale Aminbase steht,

in die enantiomerenreinen Verbindungen der allgemeinen Formel (IVa)

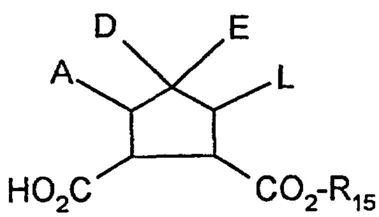

(+),(-) (IVa)

in welcher

A, D, E, L und $R^{15}$ die oben angegebene Bedeutung haben,

überführt,
anschließend nach Aktivierung der freien Carbonsäurefunktion durch Umsetzung mit flüssigem $NH_3$ die enantiomerenreinen Amide der allgemeinen Formel (V)

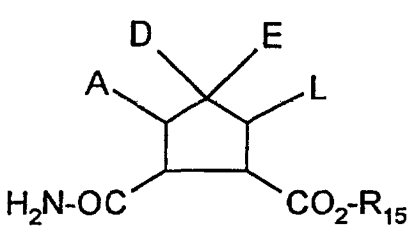

(+),(-) (V)

in welcher

A, D, E, L und $R^{15}$ die oben angegebene Bedeutung haben,

herstellt,
in einem weiteren Schritt durch Abspaltung des Restes $R^{15}$ in inerten Lösemitteln, enzymatisch oder in Anwesenheit eines Pd-Katalysators und jeweils in Abhängigkeit eines nucleophilen Hilfsmittels in die Verbindungen der allgemeinen Formel (VI) oder (VIa)

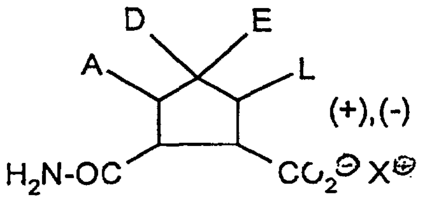
(+),(-) (VI)
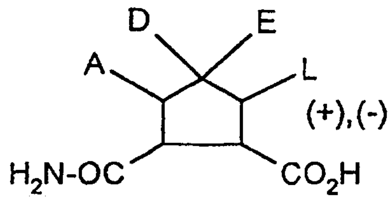
(+),(-) (VIa)

in welcher

A, D, E and L die oben angegebene Bedeutung haben,

und

X für ein Alkali- oder Erdalkaliatom steht,

überführt, und abschließend eine Hofmann-Umlagerung mit Alkali- oder Erdalkalihypochloriten in wäßriger Alkali- oder Erdalkalihydroxidlösung durchführt, in Lösung die freie Aminfunktion zunächst mit einer typischen Aminoschutzgruppe blockiert und diese nach Isolierung der geschützten Verbindungen nach üblichen Bedingungen, unter Erhalt des jeweiligen reinen Enantiomers, nach üblichen Methoden abspaltet.

2. Verfahren nach Anspruch 1, **dadurch gekennzeichnet, daß** die Verbindungen der allgemeinen Formel (V)

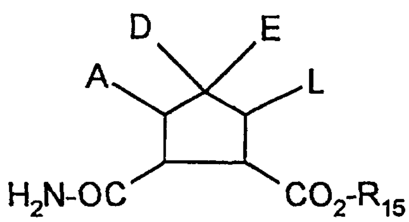
(+),(-) (V)

in welcher A, D, E, L und $R^{15}$ die in Anspruch 1 angegebene Bedeutung haben, durch Abspaltung des Restes $R^{15}$ in inerten Lösemitteln, enzymatisch oder in Anwesenheit eines Pd-Katalysators und jeweils in Abwesenheit eines nucleophilen Hilfsmittels in die Verbindungen der allgemeinen Formel (VI) oder (VIa)

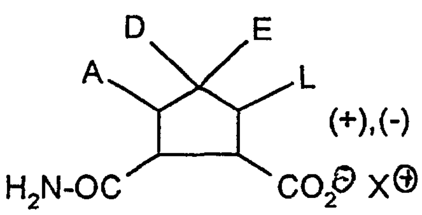
(+),(-) (VI)
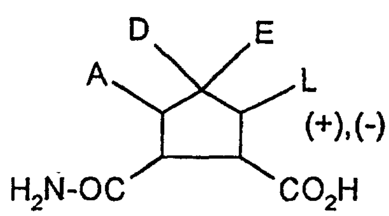
(+),(-) (VIa)

in welcher A, D, E, L and X die in Anspruch 1 angegebene Bedeutung haben, überführt werden.

3. Verfahren nach mindestens einem der Ansprüche 1 oder 2, **dadurch gekennzeichnet, dass** in den Verbindungen der Formel (I)

A, D, E und L für Wasserstoff stehen, oder

A und L für Wasserstoff stehen und

D und E gemeinsam eine Doppelbindung bilden.

**4.** Verfahren nach mindestens einem der Ansprüche 1 bis 3, **dadurch gekennzeichnet, dass** als chirale Aminbase (+), (-)-Chinin oder (+), (-)-Chinidin eingesetzt wird.

**5.** Verfahren nach mindestens einem der Ansprüche 1 bis 4, **dadurch gekennzeichnet, dass** als Pd-Katalysator Palladiumdibenzylidenaceton oder Tetrakistriphenylphosphinpalladium eingesetzt wird.

**6.** Verfahren nach mindestens einem der Ansprüche 1 bis 5, **dadurch gekennzeichnet, dass** als nucleophiles Hilfsmittel 2-Ethylhexansäure oder Natrium-2-ethyl-hexanoat eingesetzt werden.

**7.** Verfahren nach mindestens einem der Ansprüche 1 bis 6, **dadurch gekennzeichnet, dass** als Aminoschutzgruppe N-Fluorenyl-9-methoxycarbonyl eingesetzt wird.

**8.** Verfahren nach mindestens einem der Ansprüche 1 bis 7, **dadurch gekennzeichnet, dass** die Hofmann-Umlagerung mit Kaliumhypochlorit in wäßriger Kaliumhydroxidlösung durchgeführt wird.

**9.** Verfahren nach mindestens einem der Ansprüche 1 bis 8, **dadurch gekennzeichnet, dass** die Einführung der Aminoschutzgruppe zum Schutz der freien Aminfunktion in Dioxan und in Anwesenheit einer Base in einem Temperaturbereich von 0°C bis 60°C erfolgt.

**10.** Verfahren nach Anspruch 9, **dadurch gekennzeichnet, dass** als Base Natriumhydrogencarbonat eingesetzt wird.

**Claims**

**1.** Process for the preparation of enantiomerically pure cyclopentane- and -pentene β-amino acids of the general formula (I)

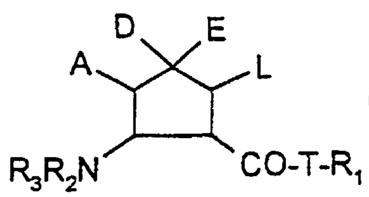

(I)

in which

A and L denote hydrogen

or

A and D or E and L in each case together form a double bond,

D and E are identical or different and represent hydrogen, halogen or hydroxyl or represent straight-chain or branched alkyl having up to 8 carbon atoms which is optionally mono- to disubstituted by identical or different substituents consisting of halogen, hydroxyl, phenyl, benzyloxy or carboxyl or of straight-chain or branched alkoxy, acyl or alkoxycarbonyl having in each case up to 6 carbon atoms or of a group of the formula $-NR^4R^5$,

in which
$R^4$ and $R^5$ are identical or different and denote hydrogen, phenyl or straight-chain or branched alkyl having up to 6 carbon atoms,

or

D and E together represent a radical of the formula

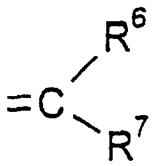

or =N-OH,
in which
$R^6$ and $R^7$ are identical or different and denote hydrogen or halogen or straight-chain or branched alkyl, alkoxy or oxyacyl having in each case up to 8 carbon atoms, or denote benzyl or phenyl,

or

D and E together represent the radical of the formula =O or =S,

$R^2$ represents hydrogen or
represents an amino-protecting group, or
represents straight-chain or branched alkyl having up to 8 carbon atoms which is optionally mono- to disubstituted by identical or different substituents consisting of hydroxyl or formyl or of straight-chain or branched acyl having up to 6 carbon atoms or of phenyl or benzoyl, which are optionally substituted up to 2 times by identical or different substituents consisting of halogen, nitro or cyano or of straight-chain or branched alkyl having up to 6 carbon atoms,
or
represents straight-chain or branched acyl having up to 8 carbon atoms,
or
represents benzoyl which is optionally substituted as described above,
or
represents a group of the formula $-SO_2R^8$,
in which

$R^8$ denotes straight-chain or branched alkyl having up to 8 carbon atoms or denotes benzyl or phenyl, the latter radicals being optionally substituted up to 3 times by identical or different substituents consisting of halogen, hydroxyl, nitro, cyano, trifluoromethyl or trifluoromethoxy or of straight-chain or branched alkyl, alkoxy or alkoxycarbonyl having in each case up to 6 carbon atoms, or carboxyl or of the above-indicated group $-NR^4R^5$,
in which

$R^4$ and $R^5$ have the meaning given above,

or

represents phenyl which is optionally substituted up to 3 times by identical or different substituents consisting of halogen, hydroxyl, nitro, trifluoromethyl, trifluoromethoxy, straight-chain or branched alkyl, acyl, alkoxy or alkoxycarbonyl having in each case up to 6 carbon atoms or of a group of the formula $-NR^4R^5$ or $-SO_2R^8$,
in which

$R^4$, $R^5$ and $R^8$ have the meaning given above,

or
represent an amino acid residue of the formula

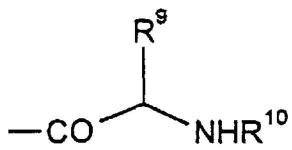

in which

$R^9$ denotes cycloalkyl having 3 to 8 carbon atoms, aryl having 6 to 10 carbon atoms or hydrogen or denotes straight-chain or branched alkyl having up to 8 carbon atoms,
the alkyl being optionally substituted by cyano, methylthio, hydroxyl, mercapto or guanidyl or by a group of the formula $-NR^{11}R^{12}$ or $R^{13}$-OC-,
in which

$R^{11}$ and $R^{12}$ independently of one another represent hydrogen, straight-chain or branched alkyl having up to 8 carbon atoms or phenyl,

and

$R^{13}$ denotes hydroxyl, benzyloxy or alkoxy having up to 6 carbon atoms or denotes the above-indicated group $-NR^{11}R^{12}$,

or the alkyl is optionally substituted by cycloalkyl having 3 to 8 carbon atoms or by aryl having 6 to 10 carbon atoms which is substituted in turn by hydroxyl, halogen, nitro or alkoxy having up to 8 carbon atoms or by the group $-NR^{11}R^{12}$,
in which

$R^{11}$ and $R^{12}$ have the meaning given above,

and

$R^{10}$ denotes hydrogen or an amino-protecting group,

$R^3$ represents hydrogen or represents straight-chain or branched alkyl having up to 8 carbon atoms which is optionally substituted by phenyl,

or

$R^2$ and $R^3$ together represent the radical of the formula $=CHR^{14}$,
in which

$R^{14}$ denotes hydrogen or straight-chain or branched alkyl having up to 8 carbon atoms which is optionally substituted by halogen, hydroxyl, phenyl or carboxyl or by straight-chain or branched alkoxy or alkoxycarbonyl having in each case up to 6 carbon atoms,

T represents an oxygen or sulphur atom or represents the -NH group,

$R^1$ represents hydrogen or represents straight-chain or branched alkyl having up to 8 carbon atoms or phenyl, the latter radicals being optionally substituted up to 3 times by identical or different substituents consisting of hydroxyl, halogen, nitro, cyano, carboxyl, trifluoromethyl or trifluoromethoxy, of straight-chain or branched alkoxy, and in the case of phenyl also of straight-chain or branched alkyl, acyl or alkoxycarbonyl having in each case up to 6 carbon atoms, or of a group of the formula $-NR^4R^5$

or -$SO^2R^8$,
in which

$R^4$, $R^5$ and $R^8$ have the meaning given above,

or, if T represents the -NH group,

$R^1$ represents the group of the formula -$SO_2R^8$,
in which

$R^8$ has the meaning given above,

**characterized in that** meso-dicarboxylic acid anhydrides of the general formula (II)

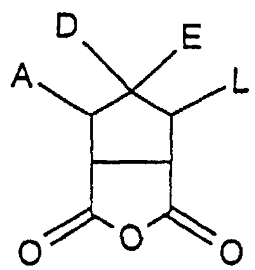

(II)

in which

A, D, E and L have the meaning given above

are converted by an asymmetric alcoholysis with alcohols of the general formula (III)

$$R^{15}\text{-OH} \quad \text{(III)}$$

in which

$R^{15}$ represents straight-chain or branched alkyl or represents alkenyl having in each case up to 5 carbon atoms, which are optionally substituted by cyano, trimethylsilyl, phenyl or trichloromethyl,

and in the presence of equimolar amounts of a chiral amine base, based on 1 mol of the dicarboxylic acid anhydrides of the general formula (II), which is present in enantiomerically pure form, in inert solvents and initially via the intermediate, enantiomerically pure salt stage of the general formula (IV)

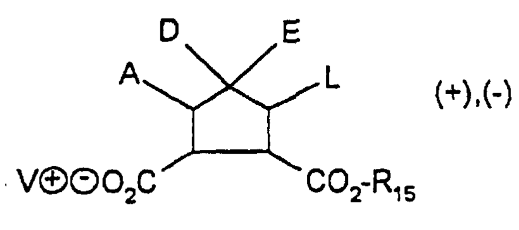

(IV)

in which

A, D, E, L and $R^{15}$ have the meaning given above

and

V  represents the chiral amine base,

to the enantiomerically pure compounds of the general formula (IVa)

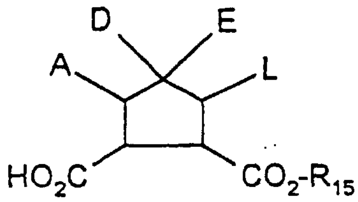

(+),(-)  (IVa)

in which

A, D, E, L and $R^{15}$  have the meaning given above,

subsequently, following activation of the free carboxylic acid function by reaction with liquid $NH_3$, the enantiomerically pure amides of the general formula (V)

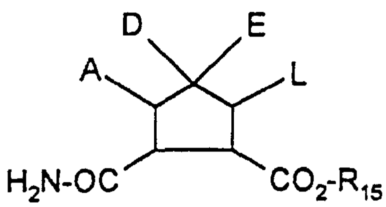

(+),(-)  (V)

in which

A, D, E, L and $R^{15}$  have the meaning given above,

are prepared,
in a further step the products are converted, by elimination of the radical $R^{15}$ in inert solvents, enzymatically or in the presence of a Pd catalyst, and in each case depending on a nucleophilic auxiliary, into the compounds of the general formula (VI) or (VIa)

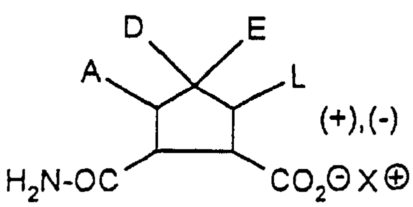

(+),(-)  (VI)

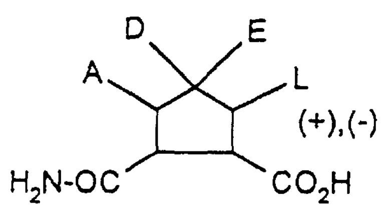

(+),(-)  (VIa)

in which

A, D, E and L  have the meaning given above,

and

X  represents an alkali metal or alkaline earth metal atom,

and finally a Hofmann rearrangement is carried out using alkali metal hypochlorites or alkaline earth metal hypochlorites in aqueous alkali metal hydroxide or alkaline earth metal hydroxide solution, the free amine function is initially blocked in solution with a typical amino-protecting group, which is eliminated by conventional methods after isolation of the protected compounds in accordance with conventional conditions to obtain the respective pure enantiomer.

**2.** Process according to Claim 1, **characterized in that** the compounds of the general formula (V)

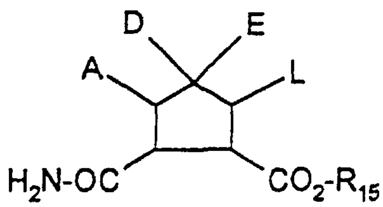

(+),(-) (V)

in which A, D, E, L and $R^{15}$ have the meaning given in Claim 1 are converted, by elimination of the radical $R^{15}$ in inert solvents, enzymatically or in the presence of a Pd catalyst, and in each case in the absence of a nucleophilic auxiliary, to the compounds of the general formula (VI) or (VIa)

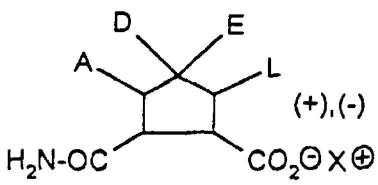

(+),(-) (VI)

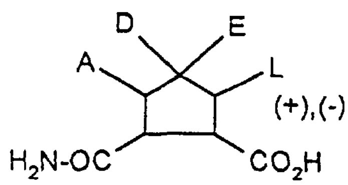

(+),(-) (VIa)

in which A, D, E, L and X have the meaning given in Claim 1.

**3.** Process according to at least one of Claims 1 or 2, **characterized in that** in the compounds of the formula (I)

| | |
|---|---|
| A, D, E and L | represent hydrogen<br>or |
| A and L | represent hydrogen<br>and |
| D and E | together form a double bond. |

**4.** Process according to at least one of Claims 1 to 3, **characterized in that** the chiral amine base used is (+),(-)-quinine or (+),(-)-quinidine.

**5.** Process according to at least one of Claims 1 to 4, **characterized in that** the Pd catalyst used is palladium dibenzylideneacetone or tetrakistriphenylphosphinepalladium.

**6.** Process according to at least one of Claims 1 to 5, **characterized in that** the nucleophilic auxiliary used is 2-ethylhexanoic acid or sodium 2-ethylhexanoate.

**7.** Process according to at least one of Claims 1 to 6, **characterized in that** the amino-protecting group used is N-fluorenyl-9-methoxycarbonyl.

**8.** Process according to at least one of Claims 1 to 7, **characterized in that** the Hofmann rearrangement is carried out using potassium hypochlorite in aqueous potassium hydroxide solution.

**9.** Process according to at least one of Claims 1 to 8, **characterized in that** the introduction of the amino-protecting group to protect the free amine function is effected in dioxane and in the presence of a base in a temperature range from 0°C to 60°C.

**10.** Process according to Claim 9, **characterized in that** the base used is sodium hydrogen carbonate.

**Revendications**

**1.** Procédé de préparation d'acides β-aminés cyclopentaniques et cyclopenténiques énantiomériquement purs de la formule générale (I) :

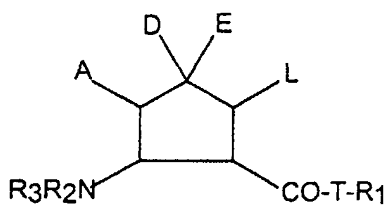

(I)

dans laquelle :

A et L représentent l'atome d'hydrogène, ou
A et D ou E et L forment chaque fois en commun, une double liaison ;
D et E sont identiques ou différents et représentent l'atome d'hydrogène, un atome d'halogène, le radical hydroxyle ou un radical alcoyle linéaire ou ramifié ayant jusqu'à 8 atomes de carbone, qui est le cas échéant, substitué 1 à 2 fois, de manière identique ou différente, par un atome d'halogène, le radical hydroxyle, phényle, benzyloxy, carboxyle ou un radical alcoxy, acyle ou alcoxycarbonyle linéaire ou ramifié, ayant chaque fois jusqu'à 6 atomes de carbone ou par un radical de formule $-NR^4R^5$,

où
$R^4$ et $R^5$ sont identiques ou différents et représentent l'atome d'hydrogène, le radical phényle ou un radical alcoyle linéaire ou ramifié ayant jusqu'à 6 atomes de carbone,
ou D et E représentent ensemble un reste de formule $=C(R^6)R^7$ ou =N-OH,
où
$R^6$ et $R^7$ sont identiques ou différents et représentent l'atome d'hydrogène, un atome d'halogène ou un radical alcoyle, alcoxy ou oxyacyle linéaire ou ramifié ayant chaque fois jusqu'à 8 atomes de carbone, le radical benzyle ou phényle,
ou D et E représentent ensemble le reste de formule =O ou =S,
$R^2$ représente l'atome d'hydrogène, ou
représente un groupe protecteur d'amino, ou
représente un radical alcoyle linéaire ou ramifié ayant jusqu'à 8 atomes de carbone, qui est le cas échéant, substitué 1 à 2 fois, de manière identique ou différente, par le radical hydroxyle, formyle, ou un radical acyle linéaire ou ramifié, ayant chaque fois jusqu'à 6 atomes de carbone ou par le radical phényle ou benzoyle, qui sont substitués le cas échéant, jusqu'à 2 fois, de manière identique ou différente, par un atome d'halogène, le radical nitro, cyano ou par un radical alcoyle linéaire ou ramifié ayant jusqu'à 6 atomes de carbone, ou
représente un radical acyle linéaire ou ramifié ayant jusqu'à 8 atomes de carbone, ou
représente le radical benzoyle qui est le cas échéant, substitué comme décrit ci-dessus, ou
représente un radical de formule $-SO_2R^8$, où
$R^8$ représente un radical alcoyle linéaire ou ramifié ayant jusqu'à 8 atomes de carbone, le radical benzyle ou phényle, où ces derniers sont le cas échéant, substitués jusqu'à 3 fois, de manière identique ou différente, par un atome d'halogène, le radical hydroxyle, nitro, cyano, trifluorométhyle, trifluorométhoxy ou par un radical alcoyle, alcoxy ou alcoxycarbonyle linéaire ou ramifié ayant chaque fois, jusqu'à 6 atomes de carbone, le radical carboxy ou par un radical $-NR^4R^5$ indiqué ci-dessus, où
$R^4$ et $R^5$ ont la signification indiquée ci-dessus, ou
représente le radical phényle, qui est le cas échéant substitué jusqu'à 3 fois, de manière identique ou différente, par un atome d'halogène, le radical hydroxyle, nitro, trifluorométhyle, trifluorométhoxy, un radical alcoyle, acyle, alcoxy ou alcoxycarbonyle linéaire ou ramifié ayant chaque fois, jusqu'à 6 atomes de carbone ou par un radical de formule $-NR^4R^5$ ou $-SO_2R^8$, où

$R^4$ $R^5$ et $R^8$ ont la signification indiquée ci-dessus, ou

représente un reste acide aminé de formule :

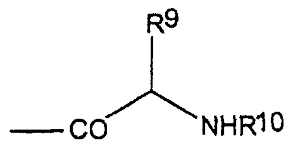

où

$R^9$ représente un radical cycloalcoyle ayant 3 à 8 atomes de carbone, aryle ayant 6 à 10 atomes de carbone ou l'atome d'hydrogène, ou un radical alcoyle linéaire ou ramifié ayant jusqu'à 8 atomes de carbone,

où le radical alcoyle est le cas échéant, substitué par le radical cyano, méthylthio, hydroxyle, mercapto, guanidyle ou par un radical de formule $-NR^{11}R^{12}$ ou $-CO-R^{13}$, où

$R^{11}$ et $R^{12}$ représentent indépendamment l'un de l'autre, l'atome d'hydrogène, un radical alcoyle linéaire ou ramifié ayant jusqu'à 8 atomes de carbone ou le radical phényle, et

$R^{13}$ représente le radical hydroxyle, benzyloxy, alcoxy ayant jusqu'à 6 atomes de carbone ou le radical $-NR^{11}R^{12}$ indiqué ci-dessus, ou

le radical alcoyle est le cas échéant, substitué par un radical cycloalcoyle ayant 3 à 8 atomes de carbone ou par un radical aryle ayant 6 à 10 atomes de carbone, qui à son tour, est substitué par le radical hydroxyle, un atome d'halogène, le radical nitro, un radical alcoxy ayant jusqu'à 8 atomes de carbone ou par le radical $-NR^{11}R^{12}$, où

$R^{11}$ et $R^{12}$ ont la signification indiquée ci-dessus,

et

$R^{10}$ représente l'atome d'hydrogène ou un radical protecteur d'amino,

$R^3$ représente l'atome d'hydrogène ou un radical alcoyle linéaire ou ramifié ayant jusqu'à 8 atomes de carbone, qui est le cas échéant, substitué par phényle, ou

$R^2$ et $R^3$ représentent ensemble, le reste de formule $=CHR^{14}$, où

$R^{14}$ représente l'atome d'hydrogène ou un radical alcoyle linéaire ou ramifié ayant jusqu'à 8 atomes de carbone, qui est le cas échéant, substitué par un atome d'halogène, le radical hydroxyle, phényle, carboxy ou par un radical alcoxy ou alcoxycarbonyle linéaire ou ramifié ayant jusqu'à 6 atomes de carbone,

T représente l'atome d'oxygène ou de soufre ou le radical NH ;

$R^1$ représente l'atome d'hydrogène, un radical alcoyle linéaire ou ramifié ayant jusqu'à 8 atomes de carbone, ou le radical phényle, où ces derniers sont le cas échéant, substitués jusqu'à 3 fois, de manière identique ou différente, par le radical hydroxyle, un atome d'halogène, le radical nitro, cyano, carboxyle, trifluorométhyle, trifluorométhoxy, par un radical alcoxy linéaire ou ramifié, dans le cas du radical phényle, également par un radical alcoyle, acyle ou alcoxycarbonyle linéaire ou ramifié, ayant chaque fois jusqu'à 6 atomes de carbone ou par un radical de formule $-NR^4R^5$ ou $-SO_2R^8$, où

$R^4$, $R^5$ et $R^8$ ont la signification indiquée ci-dessus,

ou dans le cas où T représente le radical NH,

$R^1$ représente le radical $-SO_2R^8$, où

$R^8$ a la signification indiquée ci-dessus, **caractérisé en ce que** l'on convertit l'anhydride d'acide méso-dicarboxylique de la formule générale (II) :

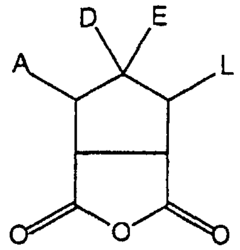

(II)

dans laquelle
A, D, E et L ont la signification indiquée ci-dessus,
par une alcoolyse asymétrique avec des alcools de la formule générale (III) :

$$R^{15}\text{-OH} \qquad \text{(III)}$$

dans laquelle :

$R^{15}$ représente un radical alcoyle ou alcényle linéaire ou ramifié, ayant chaque fois jusqu'à 5 atomes de carbone, qui sont le cas échéant, substitués par le radical cyano, triméthylsilyle, phényle ou trichlorométhyle,

et en présence de quantités équimolaires d'une base amine chirale présente sous une forme énantiomériquement pure, sur base de 1 mole de l'anhydride d'acide dicarboxylique de la formule générale (II), dans un solvant inerte, d'abord par le stade sel intermédiaire, énantiomériquement pur, de la formule générale (IV) :

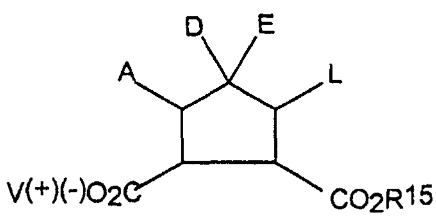

(+), (-) (IV)

dans laquelle :

A, D, E, L et $R^{15}$ ont la signification indiquée ci-dessus, et
V représente la base amine chirale ;

en le composé énantiomériquement pur de la formule générale (IVa) :

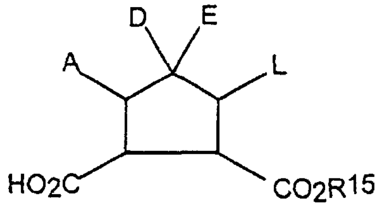

(+), (-) (IVa)

dans laquelle :

A, D, E, L et $R^{15}$ ont la signification indiquée ci-dessus,

ensuite par activation de la fonction acide carboxylique libre par réaction avec du $NH_3$ liquide, on prépare l'amide énantiomériquement pur de la formule générale (V) :

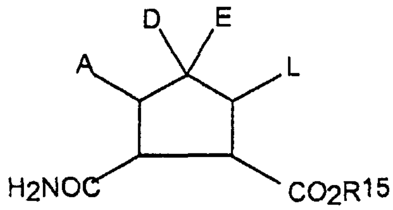

(+), (-) (V)

dans laquelle :

A, D, E, L et $R^{15}$ ont la signification indiquée ci-dessus,

dans une autre étape, on convertit par séparation du reste $R^{15}$ dans un solvant inerte, de manière enzymatique ou en présence d'un catalyseur au Pd et chaque fois, en fonction d'un auxiliaire nucléophile, en les composés de formule générale (VI) ou (VIa) :

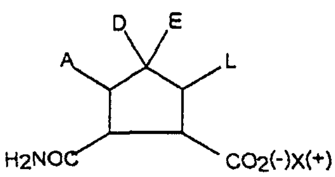

(+), (-) (VI)

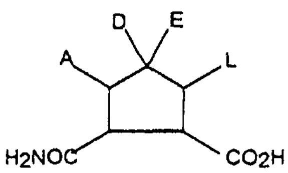

(+), (-) (VIa)

dans lesquelles :

A, D, E et L ont la signification indiquée ci-dessus, et
X représente un atome alcalin ou alcalino-terreux,

et ensuite, on réalise une transposition d'Hofmann avec un hypochlorite alcalin ou alcalino-terreux, en solution aqueuse d'hydroxyde alcalin ou alcalino-terreux, la fonction amino libre en solution est d'abord bloquée avec un groupe protecteur d'amino typique et celui-ci est enlevé par des procédés usuels, après isolement du composé protégé dans des conditions usuelles, pour obtenir l'énantiomère pur.

2. Procédé suivant la revendication 1, **caractérisé en ce que** les composés de formule générale (V) :

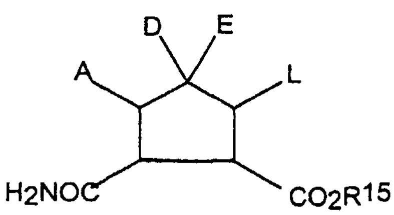

(+), (-) (V)

dans laquelle A, D, E, L et $R^{15}$ ont la signification indiquée à la revendication 1, sont transformés par enlèvement du reste $R^{15}$ dans un solvant inerte, de manière enzymatique ou en présence d'un catalyseur au Pd et chaque fois, en l'absence d'un auxiliaire nucléophile en les composés de formule générale (VI) ou (VIa) :

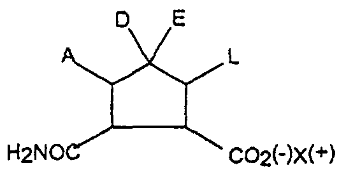 (+), (-) (VI) 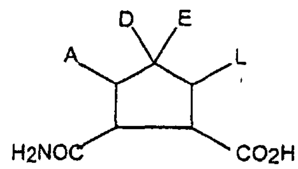 (+), (-) (VIa)

dans laquelle A, D, E, L et X ont la signification indiquée à la revendication 1.

**3.** Procédé suivant au moins l'une des revendication 1 ou 2, **caractérisé en ce que** dans les composés de formule (I),
A, D, E et L représentent l'atome d'hydrogène, ou
A et L représentent l'atome d'hydrogène, et
D et E forment ensemble une double liaison.

**4.** Procédé suivant au moins l'une des revendication 1 à 3, **caractérisé en ce que** l'on met en oeuvre comme base amine chirale, la (+), (-)-quinine ou la (+), (-)-quinidine.

**5.** Procédé suivant au moins l'une des revendication 1 à 4, **caractérisé en ce que** l'on met en oeuvre comme catalyseur au Pd, le palladium-dibenzylidèneacétone ou le tétrakistriphénylphosphine-palladium.

**6.** Procédé suivant au moins l'une des revendication 1 à 5, **caractérisé en ce que** l'on met en oeuvre comme auxiliaire nucléophile, l'acide 2-éthylhexanoïque ou le 2-éthylhexanoate de sodium.

**7.** Procédé suivant au moins l'une des revendication 1 à 6, **caractérisé en ce que** l'on met en oeuvre comme groupe protecteur d'amino, le N-fluorényl-9-méthoxycarbonyle.

**8.** Procédé suivant au moins l'une des revendication 1 à 7, **caractérisé en ce que** l'on réalise la transposition d'Hofmann avec de l'hypochlorite de potassium dans une solution aqueuse d'hydroxyde de potassium.

**9.** Procédé suivant au moins l'une des revendication 1 à 8, **caractérisé en ce que** l'introduction du groupe protecteur d'amine pour la protection de la fonction amine libre est réalisée dans le dioxanne et en présence d'une base, dans un intervalle de température allant de 0°C à 60°C.

**10.** Procédé suivant la revendication 9, **caractérisé en ce que** l'on met en oeuvre comme base, l'hydrogénocarbonate de sodium.